# EUROPEAN PATENT APPLICATION

(11) **EP 1 386 939 A1**
(43) Date of publication of application: **04.02.2004**
(21) Application number: 03016567.4
(22) Date of filing: 24.07.2003
(51) Int. Cl.: C08J 7/04, A61B 19/04, A41D 19/00, C09D 201/00

(54) **Water-based coating for rubber articles**

(30) Priority: 01.08.2002 US 211165
(71) Applicant: National Starch and Chemical Investment Holding Corporation, New Castle, Delaware 19720 (US)
(72) Inventor: Mukherjee, Apala, South Orange, New Jersey 07079 (US)
(74) Representative: Held, Stephan, Dipl.-Chem. Dr.rer.nat.

(57) **Abstract**

The present invention is directed to the use of a polymer coating composition having a high Tg polymer with a Tg of greater than -10°C and fumed silica particles for the coating on natural and synthetic rubber articles, particularly for latex gloves. The coating for rubber articles, provides a surface coating that reduces friction between the latex and the hand to allow convenient donning. The coating composition is deliverable from aqueous solution.

## Description

The present invention relates to the use of a polymeric coating composition for rubber articles. The polymer coating composition contains fumed silica particles and a high Tg polymer emulsion. The polymeric coating composition is especially useful for the inside coating of latex gloves.

### BACKGROUND OF THE INVENTION

As used herein, the terms latex glove, elastomeric material or article, and latex article refer to a glove or article made of natural or synthetic rubber. Conventional medical gloves made from natural or synthetic rubber are difficult to don without a lubricant. Generally, said gloves are manufactured with a powdered coating, such as corn starch, over the inner surface of the glove so that the gloves can be more easily put on. The powder coating is a known nuisance, as loose powder can become airborne. The powder tends to absorb proteins found in natural rubber latex and the powder is easily dislodged during donning and use, contaminating the surrounding environment and causing allergies and other negative effects. Further, the protein/powder complex serves as a food source for bacteria, allowing them to proliferate. Recently, there has been a growing demand for powder-free natural and synthetic rubber gloves, which do not use loose powder for donning and mold release.

Glove manufacturers have tried to find alternatives to using starch powder to coat gloves. Some latex glove manufacturers use a chlorination process to provide the slippage necessary to facilitate donning of the gloves. In this case, calcium carbonate is used as a mold release agent and washed away prior to chlorination. Although this reduces the tack and friction of the rubber, this process makes the rubber less pliant and reduces the shelf life of the glove.

Manufacturers have looked at polymer based coatings. To be an effective substitute for starch, the inner surface coating must not only reduce friction between the rubber and the hand to allow convenient donning, but also must allow the rubber to stretch without coating delamination, i.e. have a high coefficient of elongation combined with low tack and a low coefficient of friction. Further, the coating should be deliverable from an aqueous solution, which should be stable in extreme environmental conditions, and meet any relevant regulatory requirements.

Several types of coatings have been developed, primarily based on polyurethanes: US Patent Number 5,088,125 discloses gloves modified by an ionic polyurethane; US Patent Number 5,272,771 discloses gloves modified by an ionic polyurethane containing fully reacted isocyanate groups; and US Patent Number 5,534,350 discloses gloves in which the outer glove coating contains a polyurethane dispersion and the inside glove coating contains a polyurethane containing a silicone emulsion.

Other coatings which have been developed include emulsion copolymers, particularly core-shell, containing low surface energy monomers and hard monomers as disclosed in US Patent Number 5,700,585; or containing two monomers selected from styrene, methyl or butyl acrylates, methacrylic or acrylic acid and a silicone oligomer, with glass transition temperatures of less than 0°C and from 0 to 100°C respectively as disclosed in US Patent Number 5,712,346. These sequential emulsion polymerizations lead to substantially linear copolymers. Copending US Patent Application 09/400,488 describes the use of star polymers as coatings for latex gloves.

Other coatings have been developed containing a slip conferring component: US Patent Numbers 4,070,713 and 4,143,109 disclose a medical glove with particulate matter securely embedded in, and randomly distributed throughout the inner layer; US Patent Numbers 5,395,666, and 5,465,666 disclose a flexible article coated with a binder and porous absorbent microparticles having an oil adsorption greater than 80g or 180 g/100 g of powder. A second layer of a lubricant is then placed on top of the binder layer.

U.S. Patent Application Number 09/663,468 describes rubber articles having a coating composition of a high Tg polymer, a dispersant, and mircobeads.

U.S. Patent Number 5,691,069 describes an acrylic emulsion coating for rubber articles in which the acrylic emulsion has copolymerized within it at least one low surface energy monomer, such as a reactive silicone.

U.S. Patent number 6,016,570 describes a latex article having an intermittent polyurethane coating that contains surfactant and filler.

Surprisingly, it has now been discovered that a formulation containing a high Tg polymer and fumed silica particles provides a single layer continuous film providing an excellent slip-conferring surface deposited directly on a latex glove and other natural and synthetic rubber articles. The high Tg polymer acts as a reduced friction coating, as a binder, and as a dispersant, while the fumed silica particles provide enhanced donning properties.

### SUMMARY OF THE INVENTION

The present invention is directed to the use of a polymer coating composition having a high Tg polymer and fumed silica particles as a coating for elastomeric articles, particularly for the inner surface of latex gloves.

Other embodiments of the invention are methods of making a glove having the polymer coating composition, especially when applied to the glove as the inner glove coating.

The coating is resistant to water and can be delivered from an aqueous solution.

### DETAILED DESCRIPTION OF THE INVENTION

The present invention is directed to the use of a polymer coating composition having a high Tg polymer and fumed silica particles, as a coating for elastomeric articles. The polymer coating composition is deposited onto the elastomeric article as a single layer from an aqueous coating composition dispersion.

The high Tg polymer of the invention is a polymer or copolymer, which acts as a dispersant, as a binder, and to reduce friction. The polymer serves to distribute the individual components within the coating composition while also serving as the base coating. A high Tg polymer in the context of the invention is one having a Tg from -10 to 120°C, preferably from 25 to 110°C and most preferably from 40°C to 70°C. Polymers useful in the present invention are those formed from ethylenically unsaturated monomers or mixtures thereof, by means known in the art. Particularly useful polymers include (meth)acrylic copolymers, vinyl acrylics, polyvinyl acetate, vinyl copolymers, ethylene-vinyl acetate copolymers, and polyurethanes. In one preferred embodiment, the polymer is not a polyurethane.

The high Tg polymer can be made by means known in the art. Preferably the polymer is formed by emulsion polymerization. It is preferably present in the aqueous coating composition at from 0.1 to 30 percent by weight, preferably between 0.5 to 20 percent by weight and more preferably between 1 to 10 percent by weight, based on aqueous polymer coating composition.

The small fumed silica particles help to decrease the contact area of the rubber article, and thus reduce friction and aid donning. The fumed silica particles of the present invention are small, aggregated particles, having average particle diameters of from 1 and 200 nanometers. The hard fumed silica particles also provide anti-blocking properties to the coating. The amount of silica fillers is in the range of between 0.01 percent to 20 percent of the total coating weight, preferably between 0.05 percent to 10 percent and more preferably between 0.1 to 5 percent by weight based on aqueous polymer coating composition. The fumed silica particles form a colloidal solution when dispersed in water. The fumed silica particles of the invention provides better performance than fused silica, which is aggregated but has particle size of greater than 500 nm, and also over precipitated silica, which is nonaggregated and typically has particle size of greater than 500 nm.

Optionally, the aqueous coating composition may also contain a low surface energy material. The low energy material must be easily dispersible in a water miscible solvent and will form a single-phase aqueous dispersion with the coating composition containing a high Tg emulsion and fumed silica particles. This coating composition can be deposited on an elastomeric material to form a continuous film. Preferably the low surface energy material is present in the aqueous coating composition at between 0.01 percent to 20 percent by weight, preferably between 0.05 to 10 percent by weight and more preferably between 0.1 to 5 percent by weight, based on aqueous polymer coating composition. Examples of low surface energy materials include, but are not limited to, silicone, fluoropolymers and fatty acid esters, or mixtures thereof. A preferred low energy material is a silicone dispersion. Other preferred low surface energy materials are fluoropolymer dispersions and fatty acid ester dispersions.

In addition to the high Tg polymer and fumed silica particles, it can be advantageous to optionally add a rheology modifier to the aqueous polymer coating composition. The rheology modifier is used to control the viscosity of the composition for ease of use in different manufacturing processes and equipment. Rheology modifiers useful in the present invention include, but are not limited to cellulosics such as hyroxyethylcellulose, cationic hydroxyethylcellulose, such as Polyquaternium-4 and Polyquaternium-10, hydrophobically modified hydroxyethylcellulose, carboxymethylcellulose, methylcellulose, and hydroxypropylcellulose; dispersed or soluble starches or modified starches; and polysaccharide gums such as xanthan gum, guar gum, cationic guar gum such as Guar Hydroxypropyltrimonium Chloride, and locust bean gum. Other suitable rheology modifiers include, but are not limited to, alkali swellable emulsion polymers, which are typically made by emulsion copolymerization of (meth)acrylic acid with compatible ethylenically unsaturated monomers such as alkyl esters of (meth)acrylic acid, hydroxyalkyl esters of (meth)acrylic acid, alpha-methyl styrene, styrene, and derivatives thereof, vinyl acetate, crotonic acid, esters of crotonic acid, and acrylamide, and derivatives thereof; hydrophobically modified alkali swellable emulsion polymers, which are alkali swellable emulsion polymers into which hydrophobic groups have been introduced; certain amphiphilic polyurethanes; poly(acrylamide), copolymers of acrylamide with compatible ethylenically unsaturated monomers, poly(vinyl amides) such as poly(vinyl pyrrolidinone); and copolymers of vinyl amides such as vinyl pyrrolidinone with compatible ethylenically unsaturated monomers. The rheology modifier is typically added at from 0.01 to 1 percent by weight, and preferably from 0.05 to 0.15 percent by weight, based on the aqueous polymer coating composition.

The aqueous polymer coating composition may also contain other additives known in the art, such as adhesion promoters, surfactants, crosslinking agents, biocides, and fillers.

The polymer coating composition of the present invention is made by combining each of the ingredients to form an aqueous dispersion. For example the silica particles can be dispersed in water and then combined with the high Tg polymer. Other optional additives can be combined into the aqueous coating composition.

The aqueous polymeric coating composition may be used to coat a variety of natural and synthetic rubber items, including gloves, prophylactics, catheters, balloons, tubing, and sheeting. The aqueous composition dries to form a continuous film having 10 to 90 weight percent of high Tg polymer, 0.3 to 40 weight percent of fumed silica particles, 0 to 40 weight percent low surface energy material, and 0 to 25 weight percent of rheology modifier.

A particularly suitable end use application is the coating of latex gloves, including surgeons' gloves, physicians' examining gloves, and workers' gloves, more particularly powder-free latex gloves. The aqueous polymer coating composition may be used on the inside of the glove to form a continuous film that will provide slippage and promote donning. By continuous film, as used herein, is meant a single layer matrix that is non-intermittent and covers the surface of the article. Such a film may contain holes and cracks that are not visible without magnification.

When used to coat gloves, the aqueous polymeric coating composition may be applied using standard methods known in the art. For example, one conventional method of making latex gloves is to dip a former or mold in the shape of a hand into a coagulant mixture containing calcium nitrate. After drying, the mold is immersed in a latex emulsion for a time sufficient for the rubber to coagulate and form a coating of the desired thickness. Optionally, the glove then may be water leached to remove rubber impurities. The formed glove is then oven cured and cooled. After cooling, the glove is stripped from the mold and inverted. The gloves can then be washed off-line with water, chlorine containing water or water containing surfactants. To coat the inside of the glove, the polymer coating composition may be applied immediately before or after latex curing.

An adhesion promoter may be used, and for some polymers may be necessary, to add charge and increase the amount of polymer picked up. Such adhesion promoter is typically a water soluble salt such as sodium, calcium, zinc, or aluminum salts, particularly sodium chloride and calcium nitrate. The salt is typically provided in a concentration of up to about 40 percent, particularly from about 20 to about 40 percent by weight of coating suspension. The adhesion promoter is generally applied after leaching.

Additionally wetting agents, defoamers and surfactants may be used to enhance the film formation and properties of the coating on the latex surface. Such additives are each present in less than 20 percent of the total coating weight.

The latex article, i.e. glove, may be formed so that the polymer coating composition coats the inside surface of the article. The polymer coating composition provides the desired glove properties without the need for chlorination or other coatings, including powders. However, if only one surface is coated, chlorination or another coating may be used to provide the desired properties on the non-coated surface.

The following examples are presented to further illustrate and explain the present invention and should not be taken as limiting in any regard.
NACRYLIC 6408: (Nacan Products) Acrylic Emulsion with pH of 8.0 and 45 percent solids
AEROSIL 130, 150, 300: (Degussa Huls) Fumed Silica
Silicone Emulsion: DC-36, DC-346 (Dow Corning)
Fluoropolymer Dispersion: PTFE-30 (DuPont)
KELTROL RD: (Kelco Biopolymer) Xanthan Gum
ELVACITE 4046: (Ineos Acrylics) PolyMethyl Methacrylate

### Example 1 - Making a polymer-coated rubber glove

A ceramic mold was cleaned from contaminants, rinsed, heated to 40 to 50°C and immersed for 15 to 20 seconds into the coagulant, a 20 percent aqueous solution of calcium nitrate. After dipping into the coagulant, the coagulant-coated mold was partially dried. The mold with coagulant was then immersed into a natural rubber latex at room temperature for the time required to build up a latex deposit with a required thickness. The latex deposit was then briefly dried in the oven. The mold coated with above deposit was then leached in water at about 65°C to remove natural rubber proteins. The leached latex deposit was then dried and dipped into a polymer coating composition for up to one minute. After dipping with polymer dispersion, the latex deposit was vulcanized in the oven by heating at 90 to 130°C for 15 to 30 minutes. After vulcanization, the coated rubber article was cooled and stripped from the mold. The ceramic mold was then cleaned.

### Example 2 - Making a polymer-coated rubber glove

A ceramic mold was cleaned from contaminants, rinsed, heated to 40 to 50°C and immersed for 15 to 20 seconds into the coagulant, a 20 percent aqueous solution of calcium nitrate. After dipping into the coagulant, the coagulant-coated mold was partially dried. The mold with coagulant was then immersed into a natural rubber latex at room temperature for the time required to build up a latex deposit with a required thickness. The latex deposit was then briefly dried in the oven. The mold coated with above deposit was then leached in water at about 65°C to remove natural rubber proteins. The leached latex deposit was then vulcanized in the oven by heating at 90 to 130°C for 15 to 30 minutes. After vulcanization, the coated rubber article was again leached in water, dried and dipped into a polymer coating composition dispersion for up to one minute. After drying the polymer lubrication layer, the glove was cooled and stripped from the mold. The ceramic mold was then cleaned.

### Example 3 - Polymer Formulation

The polymer coating composition was prepared containing 2.5 percent by weight of NACRYLIC 6408 with 0.04 percent KELTROL RD, 0.2 percent silicone emulsion and 0.1 percent AEROSIL 130. The formulation was used for coating rubber articles, which exhibited excellent donning properties and good wash resistance.

### Example 4 - Polymer Formulation

The polymer coating composition was prepared containing 2.5 percent by weight of NACRYLIC 6408 with 0.1 percent silicone emulsion and 0.1 percent AEROSIL 150. The formulation was used for coating rubber articles, which exhibited good donning properties and good wash resistance.

### Example 5 - Polymer Formulation

The polymer coating composition was prepared containing 2.2 percent by weight of NACRYLIC 6408 with 0.1 percent silicone emulsion and 0.1 percent AEROSIL 300. The formulation was used for coating rubber articles, which exhibited good donning properties and good wash resistance.

### Example 6 - Polymer Formulation

The polymer coating composition was prepared containing 2.2 percent by weight of NACRYLIC 6408 with 0.1 percent fluoropolymer dispersion and 0.1 percent AEROSIL 300. The formulation was used for coating rubber articles, which exhibited good donning properties and good wash resistance.

### Example 7 - Polymer Formulation (comparative)

The polymer coating composition was prepared containing 2.5 percent by weight of NACRYLIC 6408, 0.04 percent KELTROL RD and 0.1 percent ELVACITE 4046 microbeads. The formulation formed a thick precipitate, showed limited wash resistance and had marginal donning.

### Example 8- Polymer Formulation (comparative)

The polymer coating composition was prepared containing 2.5 percent by weight of NACRYLIC 6408. 0.04 percent KELTROL RD and 0.1 percent fused silica. The formulation formed a thick precipitate did not show wash resistance and had marginal donning.

### Example 9 - Polymer Formulation (comparative)

The polymer coating composition was prepared containing 2.5 percent by weight of NACRYLIC 6408. 0.04 percent KELTROL RD, 0.2 percent DC-36 and 0.1 percent PMMA microbeads. The formulation did not show wash resistance and had marginal donning.

### Example 10 - Polymer Formulation

The polymer coating composition was prepared containing 2.5 percent by weight of NACRYLIC 6408 with 0.04 percent KELTROL RD and 0.1 percent AEROSIL 130. The formulation was used for coating rubber articles, which exhibited marginal donning properties and limited wash resistance.

### Example 11 - Polymer Formulation (comparative)

The polymer coating composition was prepared containing 2.5 percent by weight of NACRYLIC 6408 with 0.04 percent KELTROL RD and 0.1 percent silicone emulsion. The formulation was used for coating rubber articles, which exhibited marginal donning properties and good wash resistance.

## Claims

1. An article comprising a formed natural rubber or synthetic article having directly deposited thereon a continuous coating comprising:
a) 10 to 90 percent by weight of a high Tg polymer, having a Tg of greater than -10°C ;
b) 0.3 to 40 percent by weight of fumed silica particles.

2. The article of claim 1 wherein said high Tg polymer has a Tg of from +25°C to 110°C.

3. The article of claim 1, wherein the fumed silica particles are aggregated.

4. The article of claim 1, wherein the fumed silica particles have a particle size of less than 200 nm.

5. The article of claim 1, wherein the coating composition further comprises 0.1 to 40 percent by weight of a low surface energy material.

6. The article of claim 5, wherein the low surface energy material comprises a silicone, fluoropolymer, or fatty acid dispersion, or a mixture thereof.

7. The article of claim 1, wherein said coating composition further comprises 0.1 to 25 percent by weight of a rheology modifier.

8. The article of claim 1, wherein the article is selected from the group consisting of gloves, prophylactics, catheters, balloons, tubing, and sheeting.

9. The article of claim 8, wherein the article is a glove selected from the group consisting of surgeons' gloves, physicians' examining gloves, and workers' gloves.

10. The article of claim 9, wherein the glove is powder-free.

11. A method of making a glove comprising:
a) dipping a former into a liquid comprising a coagulant, removing the former from the coagulant and drying it to form a layer of coagulant on the former;
b) dipping the former into rubber latex and drying it to form a partially-cured rubber deposit on the former;
c) dipping the deposit of rubber into a dispersion comprising a high Tg polymer and fumed silica particles, and drying it to form a polymer coating on the rubber deposit;
d) vulcanizing the deposit of rubber with the polymer coating in an oven at about 100°C until the rubber is vulcanized to the desired degree and the layers are bonded to the rubber; and
e) cooling and then removing a finished glove from the said former.

12. The method of claim 11(a) wherein said liquid comprising a coagulant further comprises a mold release agent.

13. The method of claim 11, further comprising after step (b) and before step (c), dipping the partially cured rubber deposit into water for sufficient time to remove at least some soluble proteins and other contaminants from the partially cured rubber deposit to form a leached partially cured rubber deposit.

14. The method of Claim 11, where the finished articles, after removal from the formers, are further washed in water, or chlorine containing water or water containing surfactants to remove protein and other contaminants.

15. A method of making a glove comprising:
a) dipping a former into a liquid comprising a coagulant, removing the former from the coagulant and drying it to form a layer of coagulant on the former;
b) dipping the former into rubber latex and drying it to form a partially-cured rubber deposit on the former;
c) vulcanizing the deposit of in an oven at about 100°C until the rubber is vulcanized to the desired degree and the layers are bonded to the rubber;
d) dipping the deposit of rubber into a dispersion comprising a high Tg polymer and fumed silica particles, and drying it to form a polymer coating on the rubber deposit; and
e) cooling and then removing a finished glove from the said former.

16. The method of claim 15(a), wherein said liquid comprising a coagulant further comprises a mold release agent.

17. The method of claim 15, further comprising after step (b) and before step (c), dipping the partially cured rubber deposit into water for sufficient time to remove at least some soluble proteins and other contaminants from the partially cured rubber deposit to form a leached partially cured rubber deposit.

18. The method of Claim 15, where the finished articles, after removal from the formers, are further washed in water, or chlorine containing water or water containing surfactants to remove protein and other contaminants.
